# EUROPÄISCHE PATENTANMELDUNG

(11) **EP 2 740 942 A2**
(43) Veröffentlichungstag der Anmeldung: **11.06.2014**
(21) Anmeldenummer: 13005525.4
(22) Anmeldetag: 27.11.2013
(51) Int. Cl.: F04D 17/12, F04D 17/16, F04D 29/28, F04D 29/30, F04D 29/44

(54) **Medizinisches oder dentales Gebläse**

(30) Priorität: 04.12.2012 DE 102012023747
(71) Anmelder: Dürr Dental AG, 74321 Bietigheim-Bissingen (DE)
(72) Erfinder: Hägele, Andreas, D-71384 Weinstadt (DE); Schnepf, Jürgen, D-74080 Heilbronn (DE); Mauthe, Ralf, D-74348 Lauffen (DE)
(74) Vertreter: Heinrich, Hanjo

(57) **Zusammenfassung**

Ein medizinisches oder dentales Gebläse zum Absaugen oder Verdichten eines gasförmigen Arbeitsfluids, insbesondere von Luft, umfasst ein Gehäuse (12), welches einen Fluideinlass (16) und einen Fluidauslass (24) aufweist, die strömungstechnisch miteinander verbunden sind. Eine Arbeitseinheit (40) umfasst Fluidfördermittel, welche mittels eines Elektromotors (28), insbesondere mittels eines elektronisch kommutierten Elektromotors, antreibbar sind und durch welche Arbeitsfluid vom Fluideinlass (16) zum Fluidauslass (24) förderbar ist. Die Arbeitseinheit (40) ist eine Radialeinheit und die Fluidfördermittel umfassen wenigstens ein Radiallaufrad (38) mit mehreren Leitschaufeln (52) und einer zentralen Einlassöffnung (42).

## Beschreibung

Die Erfindung betrifft ein medizinisches oder dentales Gebläse zum Absaugen oder Verdichten eines gasförmigen Arbeitsfluids, insbesondere von Luft, mit
a) einem Gehäuse, welches einen Fluideinlass und einen Fluidauslass umfasst, die strömungstechnisch miteinander verbunden sind;
b) einer Arbeitseinheit, die Fluidfördermittel umfasst, welche mittels eines Elektromotors, insbesondere mittels eines elektronisch kommutierten Elektromotors, antreibbar sind und durch welche Arbeitsfluid vom Fluideinlass zum Fluidauslass förderbar ist.

Derartige Gebläse sind vom Markt her bekannt und können je nach externer Beschaltung als Saugmaschinen oder Kompressoren eingesetzt werden. Insbesondere haben sich dabei Seitenkanalgebläse etabliert.

Insbesondere im dentalen Bereich ist bei solchen Gebläsen ein hoher Wirkungsgrad und eine hohe Effizienz wünschenswert, bei denen eine hohe Gebläseleistung bei gleichzeitig günstigerem Energieverbrauch sicherstellt ist. Im dentalen Praxisbetrieb, bei dem das Gebläse zum Absaugen von Flüssigkeiten und Feststoffen dient, die am Behandlungsplatz anfallen, ist das Gebläse häufig nahe der von Mitarbeitern und Patienten genutzten Räume untergebracht. Daher ist dort insbesondere auch eine moderate Geräuschentwicklung wünschenswert.

Aufgabe der Erfindung ist, ein Gebläse der eingangs genannten Art bereitzustellen, welches in dieser Hinsicht gegenüber dem Stand der Technik verbessert ist.

Diese Aufgabe wird bei einem Gebläse der eingangs genannten Art dadurch gelöst, dass
c) die Arbeitseinheit eine Radialeinheit ist und die Fluidfördermittel wenigstens ein Radiallaufrad mit mehreren Leitschaufeln und einer zentralen Einlassöffnung umfassen.

Die Erfindung beruht auf der Erkenntnis, dass mittels eines Radialgebläses besonders effektiv verhältnismäßig hohe Unterdrücke bis zu 200 mbar erreicht werden können. Insbesondere in Verbindung mit einem elektronisch kommutierten Elektromotor steht so ein besonders effektives und energiesparendes Gebläse zur Verfügung.

Der Wirkungsgrad des Gebläses wird besonders auch durch die Strömungsführung des Arbeitsfluids durch das Gebläse beeinflusst. Der Wirkungsgrad des Gebläses ist umso höher, je ungestörter und barrierefreier der Strömungsweg des Arbeitsfluids zwischen dem Fluideinlass und dem Fluidauslass ist. Dabei muss jedoch den Möglichkeiten Rechnung getragen werden, die der im Innenraum des Gebläses vorhandene Bauraum mit den darin untergebrachten Komponenten zulässt.

Es hat sich beispielsweise als günstig erwiesen, wenn eine Leiteinrichtung vorhanden ist, über welche Arbeitsfluid zur Einlassöffnung des Radiallaufrades geleitet wird. So kann das Arbeitsfluid auf einem gezielten Strömungsweg zum Radiallaufrad geleitet werden.

Besonders von Vorteil ist es, wenn zwischen dem Anschlusselement und der Einlassöffnung des Radiallaufrades ein Ringspalt ausgebildet ist, der einen Zugang zur Einlassöffnung in einer zur Achse der Einlassöffnung parallele Richtung zulässt. Üblicherweise werden Versuche unternommen, eine solche Verbindungsstelle zu einem Laufrad durch gewinkelte, ineinander greifende Dichtstrukturen abzudichten und das Ansaugen von Falschluft aus Bereichen neben der Verbindungsstelle zu verhindern. Da ein Laufrad verdrehbar gelagert sein muss, bleibt dennoch ein Durchgangsspalt bestehen, der dann gewinkelt verläuft. In der Regel wird daher dennoch Falschluft angesaugt, die dann auf Grund des gewinkelten Durchgangsspaltes stark verwirbelt in das Laufrad gelangt und in der Strömung des Arbeitsfluids Turbulenzen erzeugt, die wiederum die Effizienz des Gebläses herabsetzen. Wenn die Falschluft jedoch parallel zum Arbeitsfluid in das Radiallaufrad einströmt, unterbleiben diese Turbulenzen; die Falschluft geht gleichsam in dem Arbeitsluftstrom auf, ohne dessen Strömung merklich zu beeinflussen.

Vorzugsweise taucht das Anschlusselement der Leiteinrichtung derart in die Einlassöffnung des Radiallaufrades ein, dass der Ringspalt zwischen einer Innenmantelfläche der Einlassöffnung des Radiallaufrades und einer Außenmantelfläche des Anschlusselements der Leiteinrichtung ausgebildet ist. Im weiteren Sinne bildet somit das Anschlusselement der Leiteinrichtung einen männlichen Teil und die Einlassöffnung des Radiallaufrades einen weiblichen Teil einer Strömungsverbindung zwischen diesen Komponenten.

Das Arbeitsfluid kann besonders effektiv von dem Radiallaufrad gefördert werden, wenn die Leiteinrichtung derart ausgebildet ist, dass das Arbeitsfluid weitgehend ohne oder nur mit einem geringen Drall an der Einlassöffnung des Radiallaufrades ankommt.

Das Arbeitsfluid wird im Radiallaufrad effektiv und ohne Strömungsstörungen aus einer axialen Strömungsrichtung in eine radiale Strömungsrichtung umgelenkt, wenn die Einlassöffnung des Radiallaufrades eine in Strömungsrichtung gekrümmte Innenmantelfläche aufweist.

Es ist dafür günstig, wenn die Einlassöffnung des Radiallaufrades durch einen Einlasskragen begrenzt ist.

Es hat sich als besonders strömungsgünstig erwiesen, wenn eine Innenmantelfläche der Einlassöffnung in Strömungsrichtung einer Kreisbahn, vorzugsweise mit einem Radius von 5 mm bis 7 mm, folgt.

Die Leitschaufeln des Radiallaufrades umfassen eine Profilnase und ein Profilende und definieren eine Skelettlinie. Strömungstechnisch ist es besonders effektiv, wenn die Leitschaufeln derart ausgebildet und angeordnet sind, dass deren Anströmwinkel an der Profilnase bezogen auf die Skelettlinie 25° bis 35°, vorzugsweise 32° bis 35° oder 25° bis 29°, beträgt. Vorzugsweise beträgt der Anströmwinkel 25°. Mit solchen Leitschaufeln arbeitet das Gebläse besonders effizient und unerwünschte Verwirbelungen an den Leitschaufeln sind verringert.

Es ist außerdem günstig, wenn die Leitschaufeln derart ausgebildet und angeordnet sind, dass deren Abströmwinkel an dem Profilende bezogen auf die Skelettlinie 37° bis 45° oder 50° bis 54° beträgt. Vorzugsweise beträgt der Abströmwinkel 40°, insbesondere, wenn der oben angesprochene Anströmwinkel 25° beträgt. Mit diesen Abströmwinkeln werden besonders verlustfreie Strömungsabrisse erreicht.

Ebenfalls zu einem gleichmäßigen Strömungsbild trägt bei, wenn die Profilnase mit einem Radius von 0,5 mm bis 2 mm verrundet ist.

Auch kann es günstig sein, wenn das Profilende mit einem Radius von 0,5 mm bis 1,0 mm verrundet ist.

Um den für die Leitschaufeln nutzbaren Bauraum des Radiallaufrades auszunutzen, ist es vorteilhaft, wenn das Radiallaufrad Leitschaufeln einer ersten Art und Leitschaufeln einer zweiten Art umfasst, wobei die Leitschaufeln der zweiten Art eine geringere Längserstreckung haben als die Leitschaufeln der ersten Art und jeweils zwischen zwei Leitschaufeln der ersten Art angeordnet sind.

Effiziente Radiallaufräder und damit ausgestattete Gebläse sind gebildet, wenn das Verhältnis des Durchmessers der Einlassöffnung zum Wirkdurchmesser des Radiallaufrades zwischen 1:10 und 1:3 beträgt, wobei der Wirkdurchmesser des Radiallaufrades durch die lichte Kontur von radial am weitesten außen liegenden Profilenden der Leitschaufeln vorgegeben ist.

Eine besonders günstige Laufradgeometrie liegt vor, wenn die Einlassöffnung einen Durchmesser von 26 mm oder 27 mm und das Radiallaufrad einen Wirkdurchmesser von 100 mm hat oder wenn die Einlassöffnung einen Durchmesser von 46 mm bis 50 mm und das Radiallaufrad einen Wirkdurchmesser von 160 mm bis 190 mm hat. Mit Blick auf ein Radiallaufrad mit größerem Wirkdurchmesser konnten besonders gute Ergebnisse mit einem Radiallaufrad erzielt werden, dessen Einlassöffnung einen Durchmesser von 46 mm hat. Beim Wirkdurchmesser konnte mit einem Wert von 160 mm ein gutes und mit einem Wirkdurchmesser von 182 mm ein besonders gutes Ergebnis erzielt werden.

Dabei erstrecken sich die Leitschaufeln bevorzugt in einem Radringbereich des Radiallaufrades, der eine konstante Dicke, vorzugsweise von 4,5 mm bis 5 mm oder von 7 mm oder von 8 mm bis 9 mm, oder eine Dicke hat, die in Richtung nach radial außen konvergiert, vorzugsweise von 7,5 mm auf 2,5 mm oder von 13 mm auf 7 mm oder von 15 mm auf 8 mm oder 9 mmm.

Nachstehend wird die Erfindung anhand von Ausführungsbeispielen anhand der Zeichnungen näher erläutert. In diesen zeigen:
- Figur 1: einen Axialschnitt eines ersten Ausführungsbeispiels einer dentalen Gebläseeinheit mit zwei Verdichterstufen, bei der an gegenüberliegenden Enden eines Rotors ein eingangsseitiges und ein ausgangsseitiges Radiallaufrad angeordnet sind;
- Figur 2: eine Draufsicht auf Leitschaufeln eines Radiallaufrades von Figur 1;
- Figur 3: einen Schnitt des Radiallaufrades von Figur 2 entlang der dortigen Schnittlinie III-III;
- Figur 4: eine Ausschnittsvergrößerung von Figur 2 gemäß dem dortigen Kreisausschnitt IV;
- Figur 5: eine Strömungsverbindung zwischen einer Leiteinrichtung und dem ausgangsseitigem Radiallaufrad der Gebläseeinheit von Figur 1 in einer Detailansicht im größeren Maßstab;
- Figur 6: einen Axialschnitt eines zweiten Ausführungsbeispiels einer dentalen Gebläseeinheit mit zwei Verdichterstufen, bei der das ausgangsseitige Radiallaufrad mit einer als Umlenkrad ausgebildeten Leiteinrichtung zusammenarbeitet;
- Figur 7: eine Explosionsansicht eines Radiallaufrades von Figur 6;
- Figur 8: eine der Figur 2 entsprechende Draufsicht auf Leitschaufeln des Radiallaufrades von Figur 7;
- Figur 9: eine perspektivische Ansicht des Umlenkrades;
- Figur 10: eine Draufsicht auf Umlenkschaufeln des Umlenkrades;
- Figur 11: einen Schnitt des Umlenkrades entlang der Schnittlinie XI-XI in Figur 10;
- Figur 12: eine Strömungsverbindung zwischen dem Umlenkrad und dem ausgangsseitigem Radiallaufrad der Gebläseeinheit von Figur 6 in einer Detailansicht im größeren Maßstab.

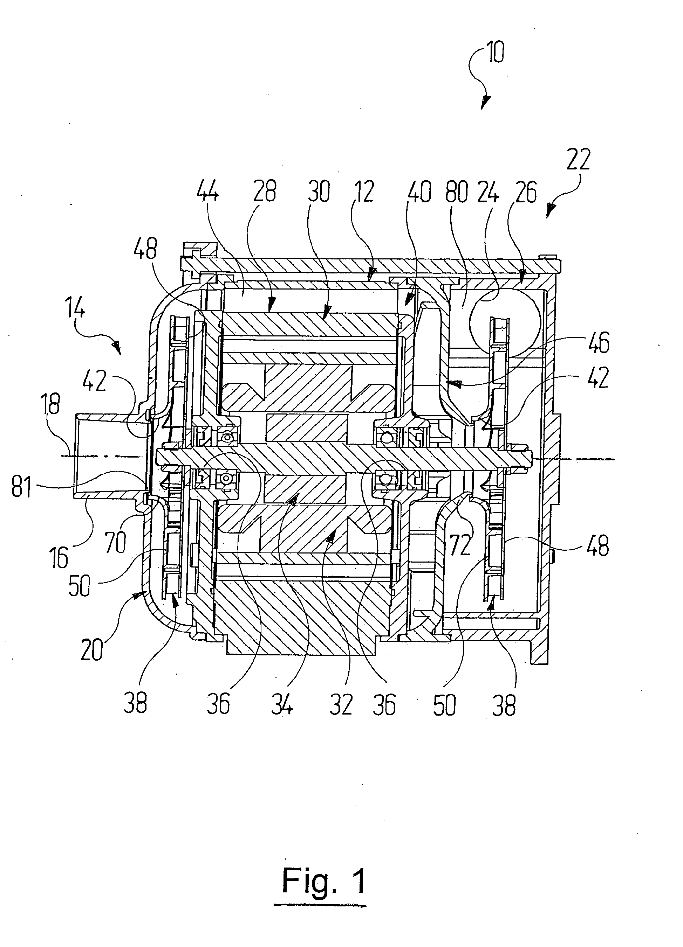

In Figur 1 ist mit 10 insgesamt eine dentale Gebläseeinheit bezeichnet, welche als Saugeinheit für einen oder mehrere dentale Behandlungsplätze verwendet wird.

Die Gebläseeinheit 10 umfasst ein mehrteiliges, insgesamt weitgehend zylindrisches Gehäuse 12. An einer Ansaugseite 14 des Gehäuses 12 befindet sich als Fluideinlass ein Einlassstutzen 16, der koaxial zur Längsachse 18 des Gehäuses 12 verläuft und Teil eines Ansaugdeckels 20 ist. An einer gegenüberliegenden Abgabeseite 22 ist als Fluidauslass ein Auslassstutzen 24 vorhanden, der sich in an und für sich bekannter Weise in radiale Richtung nach außen erstreckt und in Figur 1 lediglich als kreisrunde Durchgangsöffnung zu erkennen ist. Der Abgabestutzen 24 ist seinerseits Teil eines Abgabedeckels 26 des Gehäuses 12. Der Einlassstutzen 16 und der Auslassstutzen 24 sind strömungstechnisch miteinander verbunden.

Im Inneren des Gehäuses 12 ist ein Elektromotor 28 untergebracht, welcher seinerseits ein zylindrisches Motorgehäuse 30 umfasst, das koaxial zum Gehäuse 12 angeordnet ist. Der Elektromotor 28 ist als Innenläufermotor ausgebildet und arbeitet mit Drehzahlen zwischen 20.000 U/min und 30.000 U/min. In der Praxis ist der Elektromotor 28 ein elektronisch kommutierter Gleichstrommotor.

Bei einer nicht eigens gezeigten Abwandlung kann der Elektromotor 28 auch als Außenläufer konzipiert sein, wodurch ein größeres Drehmoment erhalten werden kann. Ein schnelleres Anlaufen des Elektromotors 28 aus dem Stillstand wird jedoch durch einen Innenläufermotor unterstützt.

Im Motorgehäuse 30 ist ein Stator 32 fixiert. Ein als Drehwelle ausgebildeter Rotor 34 erstreckt sich durch das Motorgehäuse 30 hindurch und ragt durch zwei Lageröffnungen 36 an den Stirnseiten des Motorgehäuses 30 nach außen in den Ansaugdeckel 20 bzw. den Abgabedeckel 26 hinein. Dort trägt das jeweilige Ende des Rotors 34 drehfest ein erstes, eingangseitiges Radiallaufrad 38 bzw. ein zweites, ausgangseitiges Radiallaufrad 38, welche baugleich sind und weiter unten noch im Detail erläutert werden. Die Radiallaufräder 38 bilden Fluidfördermittel einer als Radialeinheit ausgebildeten Arbeitseinheit 40. Die Radiallaufräder 38 haben eine zentrale Einlassöffnung 42 und fördern ein Arbeitsfluid vom Einlassstutzen 16 zum Auslassstutzen 24. Das Arbeitsfluid ist in der Regel Luft.

Im Betrieb der Gebläseeinheit 10 wird das Arbeitsfluid durch den Einlassstutzen 16 zur Einlassöffnung 42 des ersten Radiallaufrads 38 angesaugt und von diesem verdichtet. Das erste Radiallaufrad 38 bildet eine erste Verdichterstufe. Das Arbeitsfluid strömt durch den Ansaugdeckel 20 des Gehäuses 12 hindurch und gelangt von dort zu einem Durchgangskanal 44, der zwischen dem Gehäuse 12 und dem Motorgehäuse 30 ausgebildet ist.

Das gleichfalls vom Elektromotor 14 angetriebene zweite Radiallaufrad 38 verdichtet das vom Durchgangskanal 44 kommende Arbeitsfluid und bildet eine zweite Verdichterstufe. Vom Auslassstutzen 42 wird das Arbeitsfluid, gegebenenfalls über eine aus dem Aufstellraum der Gebläseeinheit herausführende Abgabeleitung, an die Umgebung abgegeben.

Zwischen dem Motorgehäuse 30 und dem zweiten Radiallaufrad 38 ist eine Leiteinrichtung 46 der Arbeitseinheit 40 angeordnet, welche von dem Durchgangskanal 44 kommendes Arbeitsfluid gezielt zu der Einlassöffnung 42 des zweiten Radiallaufrades 38 leitet. Dieses dient als zweite Verdichterstufe; das Arbeitsfluid strömt dann in den Abgabedeckel 26 des Gehäuses 12 und wird von dort über den Auslassstutzen 24 aus dem Gehäuse 12 heraus abgegeben.

Insgesamt ist die strömungstechnische Verbindung des Einlassstutzens 16 mit dem Auslassstutzen 24 beim vorliegenden Ausführungsbeispiel durch den Ansaugdeckel 20, den Durchgangskanal 44, die Leiteinrichtung 48 und den Abgabedeckel 26 ausgebildet.

Das durch den Durchgangskanal 44 strömende Arbeitsfluid kühlt den Elektromotor 28 an der Außenseite des Motorgehäuses 30. Ein Anteil des durch das Gehäuse 12 strömenden Arbeitsfluids kann genutzt werden, um den Elektromotor 28 außerdem zu kühlen, indem dieses Arbeitsfluid durch das Motorgehäuse 30 hindurch geführt wird, wo es am Stator 32 und am Rotor 34 vorbeiströmen und dort Wärme aufnehmen kann. Vor dem Eintritt in das Motorgehäuse 30 wird das Arbeitsfluid zweckmäßigerweise durch einen Filter geleitet, um das Arbeitsfluid beispielsweise von mitgeführten Festkörperpartikeln zu befreien.

Am Ausgang der Kühlluft aus dem Motorgehäuse 30 kann ein Schutzventil vorhanden sein, welches ein Zurückströmen von Arbeitsfluid in das Gehäuseinnere verhindert, welches nach dem Durchströmen des Motorgehäuses 30 mit Abrieb oder dergleichen verunreinigt sein kann. Auch das Eindringen von Kondensat von der Ausgangsseite der Gebläseeinheit 10 in das Motorgehäuse 30 kann so verhindert werden.

Die Gebläseeinheit 10 umfasst in an und für sich bekannter Art und Weise eine Steuereinheit, welche nicht eigens gezeigt ist und welche die Drehzahl des Elektromotors 28 und andere Betriebsparameter steuert oder regelt. In einer solchen Steuereinheit können auch verschiedene nach Wahl aktivierbare Betriebsparametersätze gespeichert sein, die unterschiedlichen Betriebsmodi der Gebläseeinheit 10 definieren.

Die Radiallaufräder 38 werden nun anhand der Figuren 1 bis 4 erläutert. Das Radiallaufrad 38 umfasst eine plane Radscheibe 48, welche nur in Figur 1 zu erkennen ist und aus einem formfesten Material wie Aluminium ausgebildet sein kann. Die Radscheibe 48 ist mit einem Schaufelteil 50 verbunden, das aus einem Kunststoff gefertigt sein kann.

Das Schaufelteil 50 umfasst die Einlassöffnung 42 und mehrere Leitschaufeln 52, welche sich von der Einlassöffnung 42 im Zentrum des Schaufelteils 50 mit einer radialen Komponente nach außen erstrecken. In den Figuren sind jeweils nur einige der Leitschaufeln 52 mit einem Bezugszeichen versehen.

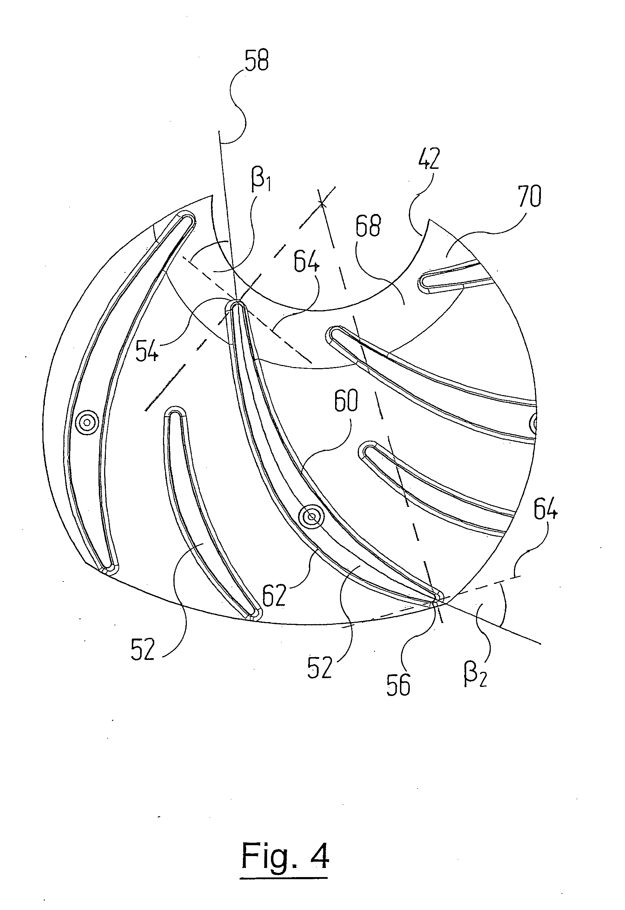

Die Leitschaufeln 52 des Radiallaufrades 38 sind gekrümmt und umfassen eine Profilnase 54 und ein Profilende 56 und definieren in an und für sich bekannter Weise eine Skelettlinie 58, welche der Übersichtlichkeit halber nur bei einer einzigen Leitschaufel 52 in Figur 4 mit einem Bezugszeichen versehen sind. Die Profilnase 54 bezeichnet dabei das jeweilige Anströmende, das zur Einlassöffnung 42 benachbart ist; das Profilende 56 bezeichnet das jeweilige Abströmende einer Leitschaufel 52, das von der Einlassöffnung 42 abliegt.

Beim vorliegenden Ausführungsbeispiel sind 16 Leitschaufeln 52 vorhanden. In der Praxis konnte mit 12 bis 18 Leitschaufeln 52 eine effiziente Förderung des Arbeitsfluids erreicht werden.

Die Leitschaufeln 52 haben eine der Einlassöffnung 42 zugewandete und gekrümmte Strömungsflanke 60 und eine von der Einlassöffnung 42 abliegende und gekrümmte Strömungsflanke 62.

Die Leitschaufeln 52 sind dabei derart ausgebildet und angeordnet, dass deren Anströmwinkel β₁ an der Profilnase 54 bezogen auf die Skelettlinie 58 32° bis 35° beträgt.

Der Anströmwinkel β₁, der auch als Schaufeleintrittswinkel bezeichnet wird, ist der Winkel zwischen der Skelettlinie 58 an der Profilnase 54 und einer gestrichelt gezeigten Bezugsgeraden 64, die einer Tangente an der Einlassöffnung 42 entspricht, die entlang einer Sekante zur Profilnase 54 hin parallelverschoben ist, wobei die Sekante durch die Skelettlinie 58 an der Profilnase 54 und durch die Mittelachse der der Einlassöffnung 42 verläuft. Dies ist anhand Figur 4 ersichtlich, wobei die Sekante mit Kurz- und Langstrichen gezeigt ist, jedoch kein Bezugszeichen trägt.

Im Hinblick auf das Profilende 56 sind die Leitschaufeln 52 derart ausgebildet und angeordnet, dass deren Abströmwinkel β₂ an dem Profilende 56 bezogen auf die Skelettlinie 58 37° bis 45° oder 50° bis 54° beträgt. Auch hier dient eine Bezugsgerade 64 als Referenz, wie es aus Figur 4 ersichtlich ist. Der Abströmwinkel wird auch als Schaufelaustrittwinkel bezeichnet.

Anströmwinkel β₁ und Abströmwinkel β₂ sind dabei in einer Bezugsebene zu betrachten, die parallel zu der Radscheibe 48 verläuft und in Figur 4 beispielsweise durch die Papierebene gebildet ist.

Die Profilnasen 54 der Leitschaufeln 52 sind mit einem Radius von 0,5 mm bis 2 mm verrundet. Das Profilende 56 der Leitschaufeln 52 ist jeweils mit einem Radius von 0,5 mm bis 1,0 mm verrundet. Die Verrundungen liegen in Ebenen parallel zur Radscheibe 48.

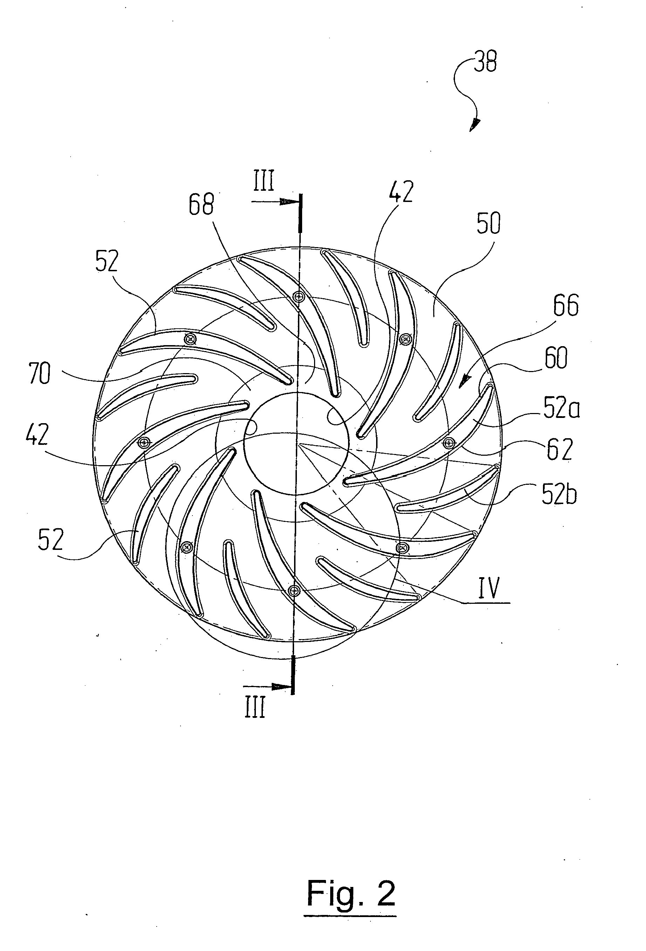

Von den Leitschaufeln 52 gibt es Leitschaufeln 52a einer ersten Art und Leitschaufeln 52b einer zweiten Art, von denen nur in Figur 2 lediglich jeweils eine Leitschaufel 52a, 52b besonders gekennzeichnet sind. Die Leitschaufeln 52b der zweiten Art haben eine geringere Längserstreckung als die Leitschaufeln 52a der ersten Art und sind jeweils zwischen zwei Leitschaufeln 52a der ersten Art angeordnet, wie es in Figur 2 gut zu erkennen ist. Die obigen geometrischen Angaben zu Leitschaufeln 52 gelten für die Leitschaufeln 52a, 52b beider Arten.

Im Hinblick auf seine Dimensionen ist das Radiallaufrad 38 derart ausgebildet, dass das Verhältnis des Durchmessers der Einlassöffnung 42 zum Wirkdurchmesser des Radiallaufrades 38 zwischen 1:10 und 1:3 beträgt. Der Wirkdurchmesser des Radiallaufrades 38 ist dabei durch die lichte Kontur von radial am weitesten außen liegenden Profilenden 56 der Leitschaufeln 52 vorgegeben. Beispielsweise können die Radscheibe 48 und das Schaufelteil 50 auch über diesen Wirkdurchmesser überstehen.

Beim vorliegenden Ausführungsbeispiel hat die Einlassöffnung 42 einen Durchmesser von 26 mm oder 27 mm und das Radiallaufrad einen Wirkdurchmesser von 100 mm.

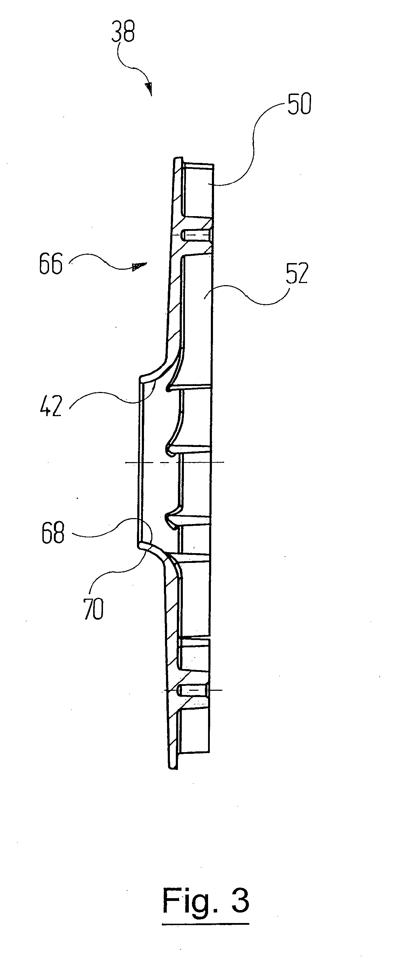

Wie in Figur 3 zu erkennen ist, erstrecken sich die Leitschaufeln 52 beim vorliegenden Ausführungsbeispiel in einem Radringbereich 66 des Radiallaufrades 38, der eine konstante Dicke hat. Diese Dicke kann zwischen 4,5 mm bis 5 mm liegen.

Anhand von Figur 3 ist ebenfalls gut zu erkennen, dass die Einlassöffnung 42 des Radiallaufrades 38 eine in Strömungsrichtung gekrümmte Innenmantelfläche 68 aufweist. Ingesamt ist die Einlassöffnung 42 durch einen Einlasskragen 70 begrenzt, der sich in axialer Richtung des Radiallaufrades 38 erstreckt. Die Leitschaufeln 52 stehen mit ihrem Endbereich an der Profilnase 54 der Innenmantelfläche 68, was besonders in Figur 3 gut zu erkennen ist.

Die Innenmantelfläche 68 der Einlassöffnung 42 folgt in Strömungsrichtung einer Kreisbahn, was der Schnitt nach Figur 3 veranschaulicht. In der Praxis konnte bei Radien dieser Kreisbahn von 5 mm bis 7 mm gute Förderergebnisse erzielt werden.

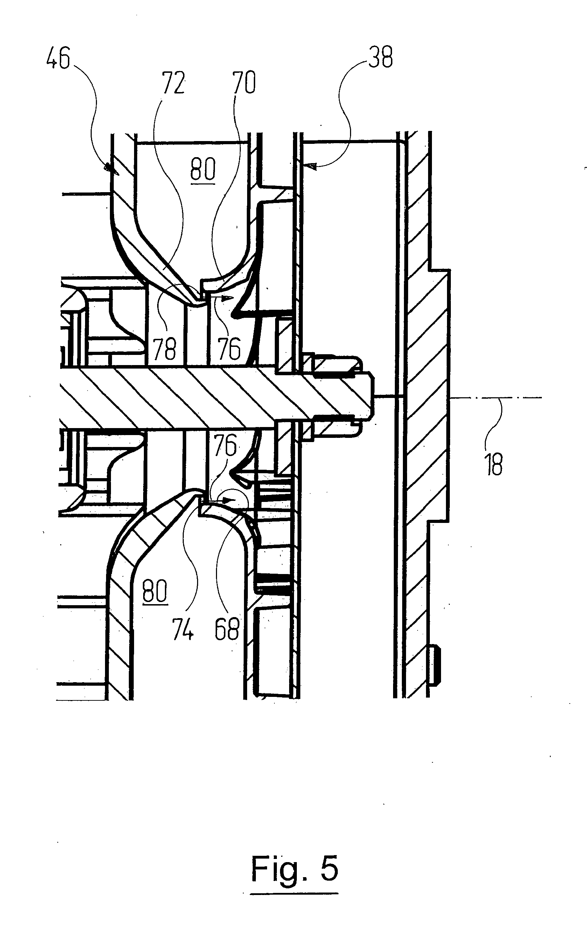

In Figur 5 ist die Strömungsverbindung zwischen der Leiteinrichtung 46 und dem ausgangsseitigen Radiallaufrad 38 der Gebläseeinheit 10 nochmals in größerem Maßstab gezeigt. Wie dort zu erkennen ist, umfasst die Leiteinrichtung 46 ein Anschlusselement in Form eines Ringstutzens 72. Zwischen dem Ringstutzen 72 der Leiteinrichtung 46 und der Einlassöffnung 42 des Radiallaufrades 38 ist ein Ringspalt 74 ausgebildet ist, der einen Zugang zur Einlassöffnung 42 in einer zur Achse der Einlassöffnung 42 parallelen Richtung zulässt, die hier mit der Achse 18 zusammenfällt und kein eigenes Bezugszeichen trägt. Dieser Zugang ist in Figur 5 durch Pfeile 76 veranschaulicht.

Dabei taucht der Ringstutzen 72 der Leiteinrichtung 46 derart in die Einlassöffnung 42 des Radiallaufrades 38 ein, dass der Ringspalt 74 zwischen der Innenmantelfläche 68 der Einlassöffnung 42 des Radiallaufrades 38 und einer Außenmantelfläche 78 des Ringstutzens 72 der Leiteinrichtung 46 ausgebildet ist.

Im Betrieb der Gebläseeinheit wird so Luft aus einem Bereich 80 zwischen der Leiteinrichtung 46 und dem Radiallaufrad 38 in das Radiallaufrad 38 angesaugt. Es kommt im Ringspalt 74 jedoch nicht zu Verwirbelungen, welche eine gleichmäßige Strömung des Arbeitsfluids stören und die Effizienz der Gebläseeinheit 10 besonders an der zweiten Verdichterstufe herabsetzen würden.

Eine derartige Strömungsverbindung ist auch zwischen dem Einlassstutzen 16 und dem ersten, eingangsseitigen Radiallaufrad 38 ausgebildet. Hierzu umfasst der Ansaugdeckel 20 eine Ringschürze 81, welche den Ansaugstutzen 16 weiterführt und unter Ausbildung eines ebenfalls mit 74 bezeichneten Ringspaltes in die Einlassöffnung 42 des ersten, eingangsseitigen Radiallaufrades 38 eintaucht.

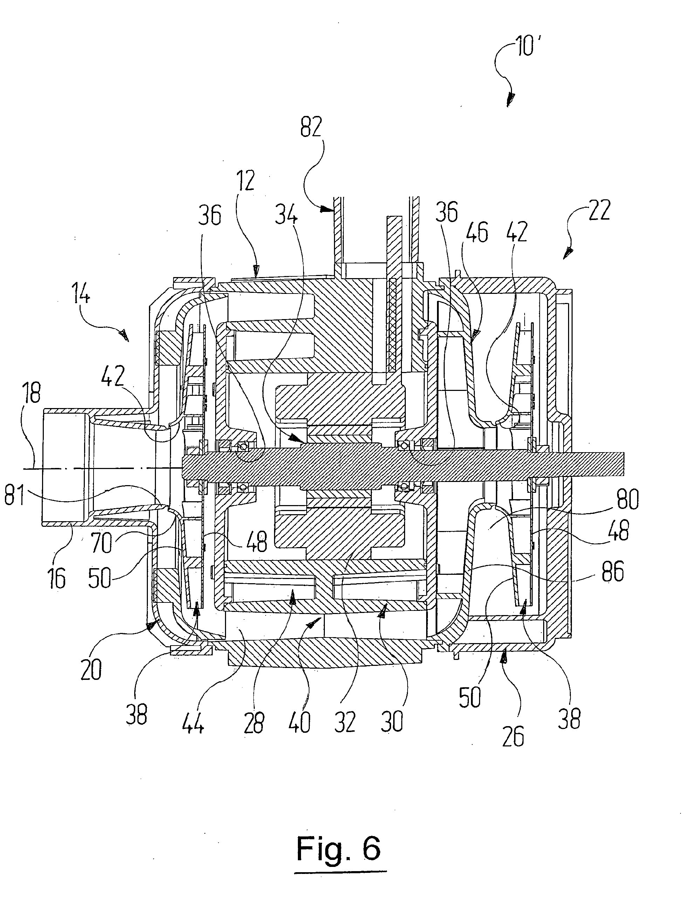

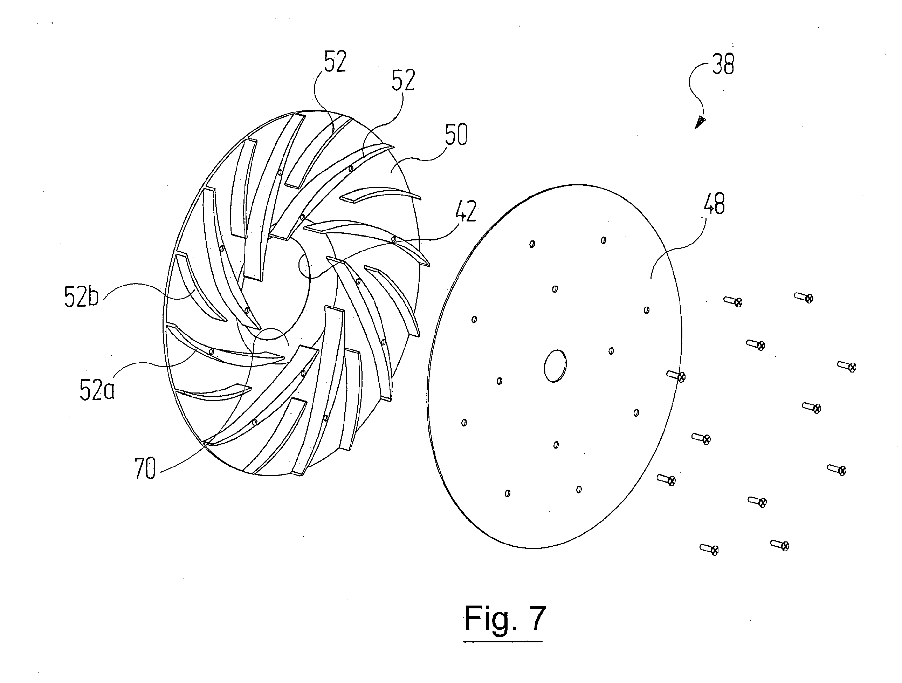

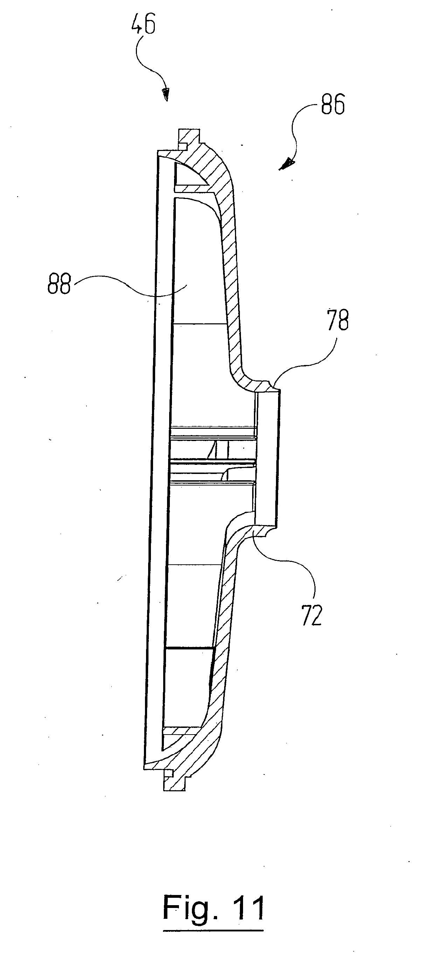

Figur 6 zeigt eine Gebläseeinheit 10', welche für den Klinikbetrieb konzipiert ist und von welcher Details in den Figuren 7 bis 12 gezeigt sind. Komponenten und Bauteile gleicher Funktion, die bereits oben erläutert wurden, sind jeweils mit denselben Bezugszeichen versehen. Der Auslassstutzen 24 ist dort auf Grund des Schnittes nicht zu sehen.

Am Gehäuse 12 ist in Figur 6 oben ein Verbindungssteg 82 zu erkennen, an dem ein nicht eigens gezeigtes Steuergehäuse für die oben angesprochene Steuereinheit befestigt ist.

Die Klinikgebläseeinheit 10' entspricht dem Arbeitsprinzip der Gebläseeinheit 10 nach den Figuren 1 bis 5, ist jedoch in Details demgegenüber abgewandelt.

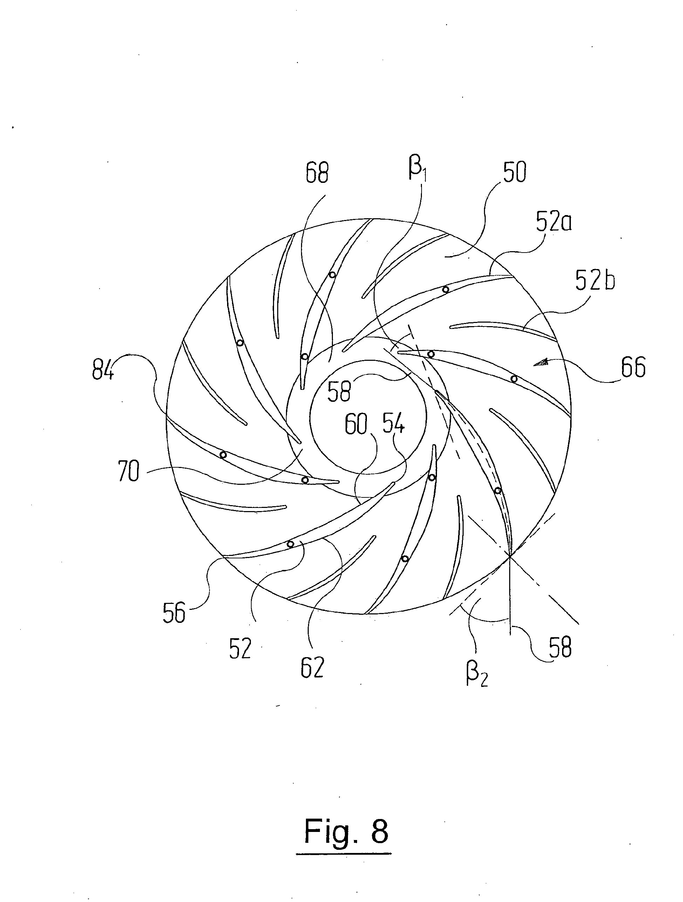

So zeigen die Figuren 7 und 8 ein abgewandeltes Radiallaufrad 38, bei welchem die Leitschaufeln 52 im Hinblick auf den Anströmwinkel β₁ derart ausgebildet und angeordnet sind, dass deren Anströmwinkel β₁ an der Profilnase 54 bezogen auf die Skelettlinie 58 25° bis 29° beträgt. Im Hinblick auf die Anströmwinkel β₁ können allgemein gute Strömungsergebnisse erzielt werden, wenn diese zwischen 25° und 35° betragen. Dies gilt auch mit Bezug auf das Radiallaufrad 38 nach den Figuren 1 bis 5.

Außerdem sind die Leitschaufeln 52 beim Radiallaufrad 38 nach den Figuren 7 und 8 im Hinblick auf den Abströmwinkel β₂ derart ausgebildet und angeordnet, dass deren Abströmwinkel β₂ an dem Profilende 56 bezogen auf die Skelettlinie 58 zwischen 40° und 54° beträgt (vgl. Figur 8), wobei mit Abströmwinkeln β₂ zwischen 40° und 45°, und dabei besonders von 40°, oder zwischen 50° bis 54° gute Ergebnisse erzielt wurden.

Gute Ergebnisse wurden insbesondere bei einem Anströmwinkel β₁ von 25° und einem Abströmwinkel β₂ von 40° erreicht. Diese Winkel kamen besonders bei einem Wirkdurchmesser von 182 mm zu Tragen.

Figur 7 veranschaulicht dabei zudem das oben erläuterte Konstruktionsprinzip der Radiallaufräder 38. Wie dort zu erkennen ist, können die Radscheibe 48 und das Schaufelteil 50 miteinander verschraubt sein. Die Schrauben greifen dabei in die Leitschaufeln 52 ein.

Wie in Figur 8 zu erkennen ist, sind die Profilenden 56 der Leitschaufeln 52 der Klinikgebläseeinheit 10' nicht verrundet, sondern weisen eine scharfe Abrisskante 84 auf. Dort weisen die Leitschaufeln 52 eine Dicke von 0,5 mm bis 1,0 mm auf. Die Schaufelzahl beträgt hier wieder 16, wobei in der Praxis mit 18 bis 22 Leitschaufeln 52 gute Ergebnisse erzielt werden konnten.

Im Gegensatz zu dem Radiallaufrad 38 der Gebläseeinheit 10 nach den Figuren 1 bis 5 ist der Radringbereich 66 hier nicht weitgehend konstant dick, sondern hat eine Dicke, die in Richtung nach radial außen konvergiert. Beim vorliegenden Ausführungsbeispiel von 7,5 mm auf 2,5 mm. Bei einer nicht eigens gezeigten Abwandlung konvergiert die Dicke von 13 mm auf 7 mm oder von 15 mm auf 8 mm oder 9 mm. Aber auch eine konstante Dicke kann vorgesehen sein, die dann z.B. 7 mm oder zwischen 8 mm und 9 mm beträgt.

Der Wirkdurchmesser der Radiallaufräder 38 bei der Klinikgebläseeinheit 10' beträgt zwischen 160 mm und 190 mm, die Einlassöffnung 42 hat einen Durchmesser zwischen 46 mm und 50 mm. In der Praxis konnten mit einem Wirkdurchmesser von 160 mm und besonders mit einem Wirkdurchmesser von 182 mm besonders gute Ergebnisse erzielt werden.

Ansonsten gilt das oben zu den Radiallaufrädern 38 nach den Figuren 1 bis 5 Gesagte sinngemäß entsprechend.

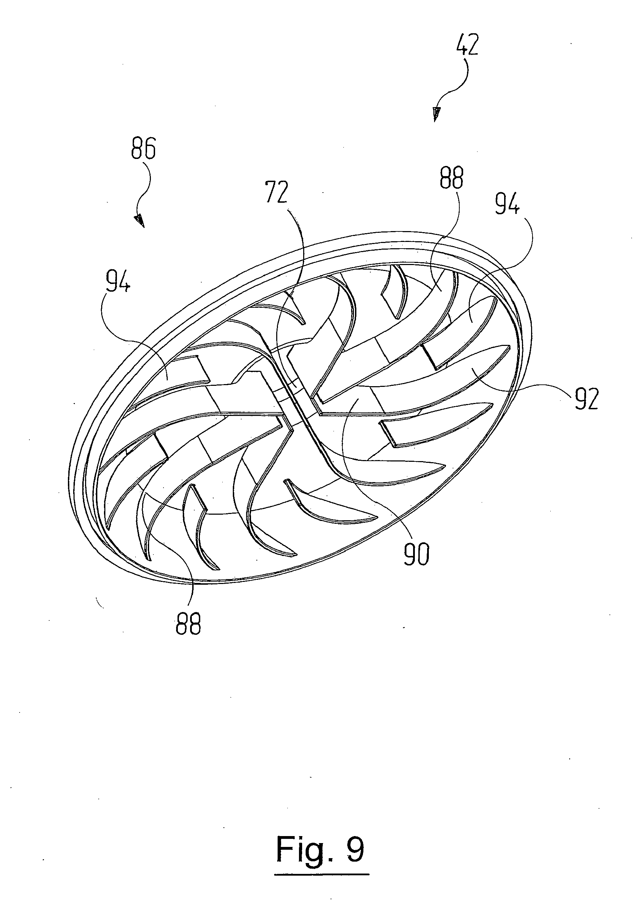

Die Figuren 9 bis 11 zeigen die Leiteinrichtung 46 im Detail, die in dieser Form auch bei der Gebläseeinheit 10 nach den Figuren 1 bis 5 vorhanden sein kann.

Die Leiteinrichtung 46 ist als Umlenkrad 86 ausgebildet, welches im Betrieb der Klinikgebläseeinheit 10' jedoch stationär verbleibt und gehäusefest verankert ist. Das Umlenkrad 86 umfasst den Ringstutzen 72 und Umlenkmittel in Form von mehreren Umlenkschaufeln 88 auf der gegenüberliegenden Seite des Ringstutzens 72, die sich von dessen Mittelachse radial nach außen erstrecken. Die Umlenkschaufeln 88 sind als flache Stege ausgebildet und umfassen von innen nach außen einen geradlinigen Abschnitt 90, der in einen gekrümmten Abschnitt 92 übergeht, der annähernd einen Viertelkreis beschreibt.

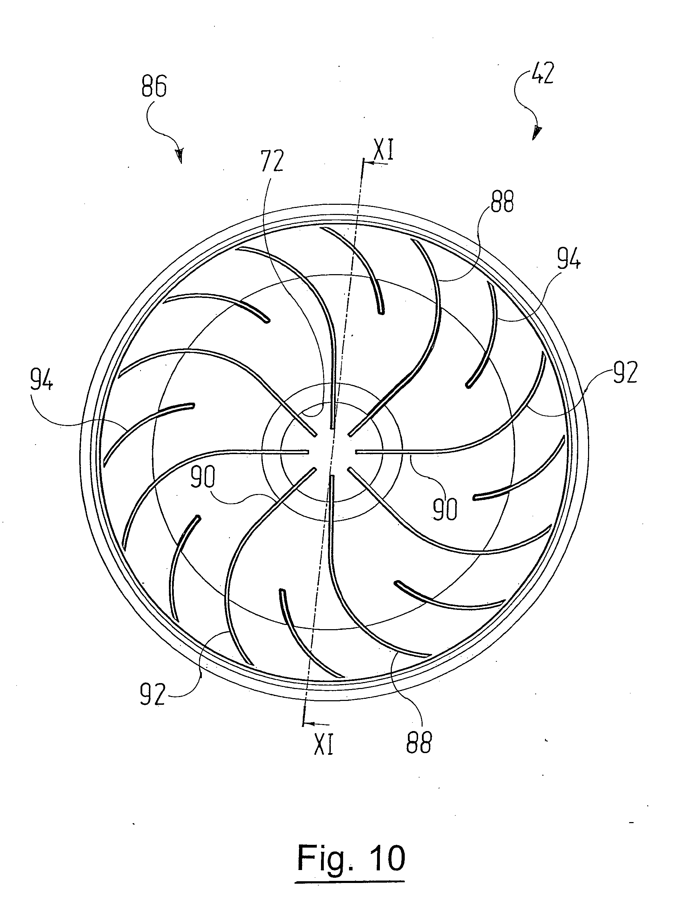

Ähnlich wie bei den Leitschaufeln 52 sind die Umlenkschaufeln 88 solche einer ersten Art und es gibt Umlenkschaufeln 94 einer zweiten Art, die eine geringere Längserstreckung als die Umlenkschaufeln 88 der ersten Art haben und die jeweils zwischen zwei Umlenkschaufeln 88 der ersten Art angeordnet sind, wie es in Figur 10 gut zu erkennen ist.

Das Umlenkrad 86 ist allgemein derart ausgebildet, dass das Arbeitsfluid ohne oder nur mit einem geringen Drall an der Einlassöffnung 42 des zweiten Radiallaufrades 38 ankommt, nachdem es aus dem Durchgangskanal 44 zwischen dem Motorgehäuse 30 und dem Gehäuse 12 ausströmt. Als geringer Drall wird beispielsweise ein Drall von 5° verstanden. Der gekrümmte Abschnitt 92 einer Umlenkschaufel 88 ist dabei derart ausgebildet, dass das Arbeitsfluid aus dem Durchgangskanal 44 in einem Winkel von 15° bis 25° auf die Umlenkschaufel 88 auftrifft.

Insgesamt sind beim vorliegenden Ausführungsbeispiel 16 Umlenkschaufeln 88 und 94 vorhanden. In der Praxis konnten mit 14 bis 18 Umlenkschaufeln 88, 94 gute Umlenkergebnisse erzielt werden.

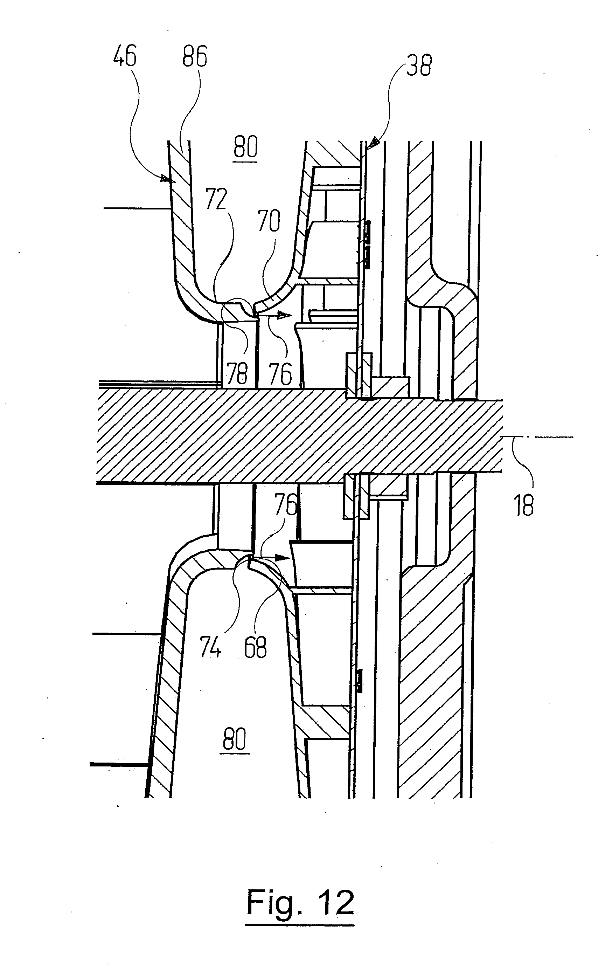

Figur 12 zeigt den Strömungsübergang von dem Umlenkrad 86 zum zweiten Radiallaufrad 38 in größerem Maßstab und zeigt wieder den Ringspalt 74 zwischen der Innenmantelfläche 68 des Einlasskragens 70 des Radiallaufrades 38 und der Außenmantelfläche 78 des Ringstutzens 72 des Umlenkrades 86, durch den störende Verwirbelungen beim Eintritt des Arbeitsfluids in das zweite, ausgangsseitige Radiallaufrad 38 unterbleiben.

Auch bei dem Klinikgebläse 10' folgt der Strömungsübergang von dem Einlassstutzen 16 zum ersten, eingangsseitigen Radiallaufrad 38 diesem Konzept, was in Figur 7 gezeigt ist.

Die Gebläseeinheiten 10 und 10' können auch als Verdichter eingesetzt werden, indem beispielsweise über den Einlassstutzen 16 Umgebungsluft angesaugt und die am Auslassstutzen 24 erhaltene verdichtete Luft einem Sammelbehälter zugeführt wird.

Durch die oben erläuterten Ausbildungen der Komponenten der Gebläseeinheiten 10 und 10' sind diese besonders effizient und energiesparend. Strömungstechnisch konnten Wirkungsgrade von 40% bis 50% erreicht werden, wodurch der Wirkungsgrad bekannter Seitenkanalmaschinen, die nicht als Radialgebläse ausgebildet sind, um bis zu 60% gesteigert werden konnte. Zudem kann ein guter Wirkungsgrad über einen breiteren Drehzahlbereich aufrechterhalten werden, als es aus dem Stand der Technik bekannte ist.

Die Radiallaufräder 38 führen durch die strömungstechnischen Maßnahmen, d.h. durch die Ausbildung der Einlassöffnung 42 und der Leitschaufeln 52 sowie durch die umgesetzten Dimensionen und Anordnungen, zu der erforderlichen Saugleistung bei relativ niedrigem Energieverbrauch.

Auch der jeweilige Strömungsübergang zu den Radiallaufrädern 38 mit den Ringspalten 74 trägt zu der erreichten Effizienz bei.

Außerdem beeinflusst das Verhältnis des Durchmessers der Einlassöffnung 42 zum Wirkdurchmesser des jeweiligen Radiallaufrades 38, das vorliegend zwischen 1:10 und 1:3 beträgt, den erhaltbaren Wirkungsgrad positiv.

Wenn die oben erläuterten Gebläseeinheiten 10 und 10' in der dentalen Praxis betrieben werden, ist deren Ansaugseite 14 in der Regel mit der Ausgangsseite eines Separators verbunden, durch welchen ein vom Behandlungsplatz kommendes Gemisch aus Luft, Flüssigkeit und Feststoffen getrennt wird, so dass nur die Luft zu der Gebläseeinheit 10 oder 10' gelangt. Für einen einwandfreien Betrieb der Gebläseeinheiten 10 oder 10' ist es wichtig, dass keine Flüssigkeit oder gar Feststoffe angesaugt werden. Daher ist in diesem Fall eine Sicherheitseinrichtung vorhanden, durch welche verhindert wird, dass das Gebläse 10 oder 10' betrieben werden kann, wenn der vorgeschaltete Separator inaktiv ist. Anders ausgedrückt, kann das Gebläse 10 oder 10' nur betrieben werden, wenn der vorgeschaltete Separator aktiviert ist.

Beispielsweise kann ein Drehfrequenzsignal am Motor des Separators abgenommen werden; wenn kein Signal vorliegt, steht der Separator still und das Gebläse 10 oder 10' wird gesperrt. Auf diese Weise kann auch überwacht werden, ob die Drehzahl des Separators ausreichend hoch ist. Bei einer zu geringen Drehzahl kann die Abscheidewirkung zu gering sein und es besteht die Gefahr, dass Flüssigkeit und/oder Feststoffe in das Gebläse 10, 10' gelangen. Daher kann der Betrieb des Gebläses 10 oder 10' beispielsweise auch bereits dann zur Sicherheit gesperrt werden, wenn die Drehzahl des Separators einen vorgegebenen, unteren Schwellenwert unterschreitet.

Ein geeignetes Drehfrequenzsignal kann beispielsweise mittels eines an und für sich bekannten Hall-Sensors abgenommen werden, der mit einem Magneten zusammenarbeitet. Dieser kann beispielsweise an einer Drehwelle oder einem Lüfter des Separatormotors angebracht sein.

## Patentansprüche

1. Medizinisches oder dentales Gebläse zum Absaugen oder Verdichten eines gasförmigen Arbeitsfluids, insbesondere von Luft, mit
a) einem Gehäuse (12), welches einen Fluideinlass (16) und einen Fluidauslass (24) umfasst, die strömungstechnisch miteinander verbunden sind;
b) einer Arbeitseinheit (40), die Fluidfördermittel umfasst, welche mittels eines Elektromotors (28), insbesondere mittels eines elektronisch kommutierten Elektromotors, antreibbar sind und durch welche Arbeitsfluid vom Fluideinlass (16) zum Fluidauslass (24) förderbar ist,
**dadurch gekennzeichnet, dass**
c) die Arbeitseinheit (40) eine Radialeinheit ist und die Fluidfördermittel wenigstens ein Radiallaufrad (38) mit mehreren Leitschaufeln (52) und einer zentralen Einlassöffnung (42) umfassen.

2. Gebläse nach Anspruch 1, **dadurch gekennzeichnet, dass** eine Leiteinrichtung (46) vorhanden ist, über welche Arbeitsfluid zur Einlassöffnung (42) des Radiallaufrades (38) geleitet wird.

3. Gebläse nach Anspruch 2, **dadurch gekennzeichnet, dass** die Leiteinrichtung (46) ein Anschlusselement (72) umfasst, wobei zwischen dem Anschlusselement (72) und der Einlassöffnung (42) des Radiallaufrades (38) ein Ringspalt (74) ausgebildet ist, der einen Zugang (76) zur Einlassöffnung (42) in einer zur Achse (18) der Einlassöffnung (42) parallele Richtung zulässt.

4. Gebläse nach Anspruch 3, **dadurch gekennzeichnet, dass** das Anschlusselement (72) der Leiteinrichtung (46) derart in die Einlassöffnung (42) des Radiallaufrades 838) eintaucht, dass der Ringspalt (74) zwischen einer Innenmantelfläche (68) der Einlassöffnung (42) des Radiallaufrades (38) und einer Außenmantelfläche (78) des Anschlusselements (72) der Leiteinrichtung (46) ausgebildet ist.

5. Gebläse nach einem der Ansprüche 2 bis 4, **dadurch gekennzeichnet, dass** die Leiteinrichtung (46) derart ausgebildet ist, dass das Arbeitsfluid weitgehend ohne oder nur mit einem geringen Drall an der Einlassöffnung (42) des Radiallaufrades (38) ankommt.

6. Gebläse nach einem der Ansprüche 1 bis 5, **dadurch gekennzeichnet, dass** die Einlassöffnung (42) des Radiallaufrades (38) eine in Strömungsrichtung gekrümmte Innenmantelfläche (68) aufweist.

7. Gebläse nach einem der Ansprüche 1 bis 6, dass die Einlassöffnung (42) des Radiallaufrades (38) durch einen Einlasskragen (70) begrenzt ist.

8. Gebläse nach einem der Ansprüche 1 bis 7, **dadurch gekennzeichnet, dass** eine Innenmantelfläche (68) der Einlassöffnung (42) in Strömungsrichtung einer Kreisbahn, vorzugsweise mit einem Radius von 5 mm bis 7 mm, folgt.

9. Gebläse nach einem der Ansprüche 1 bis 8, **dadurch gekennzeichnet, dass** jede Leitschaufel (52) des Radiallaufrades (38) eine Profilnase (54) und ein Profilende (56) umfasst und eine Skelettlinie (58) definiert.

10. Gebläse nach Anspruch 9, **dadurch gekennzeichnet, dass** die Leitschaufeln (52) derart ausgebildet und angeordnet sind, dass deren Anströmwinkel (□_{□}) an der Profilnase (54) bezogen auf die Skelettlinie (58) 25° bis 35°, vorzugsweise 32° bis 35° oder 25° bis 29°, beträgt.

11. Gebläse nach Anspruch 9 oder 10, **dadurch gekennzeichnet, dass** die Leitschaufeln (52) derart ausgebildet und angeordnet sind, dass deren Abströmwinkel (□_{□}) an dem Profilende (56) bezogen auf die Skelettlinie (58) 37° bis 45° oder 50° bis 54° beträgt.

12. Gebläse nach einem der Ansprüche 9 bis 11, **dadurch gekennzeichnet, dass** die Profilnase (54) mit einem Radius von 0,5 mm bis 2 mm verrundet ist.

13. Gebläse nach einem der Ansprüche 9 bis 12, **dadurch gekennzeichnet, dass** das Profilende (56) mit einem Radius von 0,5 mm bis 1,0 mm verrundet ist.

14. Gebläse nach einem der Ansprüche 9 bis 13, **dadurch gekennzeichnet, dass** das Radiallaufrad (38) Leitschaufeln (52a) einer ersten Art und Leitschaufeln (52b) einer zweiten Art umfasst, wobei die Leitschaufeln (52b) der zweiten Art eine geringere Längserstreckung haben als die Leitschaufeln (52a) der ersten Art und jeweils zwischen zwei Leitschaufeln (52a) der ersten Art angeordnet sind.

15. Gebläse nach einem der Ansprüche 1 bis 14, **dadurch gekennzeichnet, dass** das Verhältnis des Durchmessers der Einlassöffnung (42) zum Wirkdurchmesser des Radiallaufrades zwischen 1:10 und 1:3 beträgt, wobei der Wirkdurchmesser des Radiallaufrades (38) durch die lichte Kontur von radial am weitesten außen liegenden Profilenden (56) der Leitschaufeln (52) vorgegeben ist.

16. Gebläse nach Anspruch 15, **dadurch gekennzeichnet, dass**
a) die Einlassöffnung (42) einen Durchmesser von 26 mm oder 27 mm und das Radiallaufrad (38) einen Wirkdurchmesser von 100 mm hat;
oder
b) die Einlassöffnung (42) einen Durchmesser von 46 mm bis 50 mm und das Radiallaufrad (38) einen Wirkdurchmesser von 160 mm bis 190 mm hat.

17. Gebläse nach einem der Ansprüche 1 bis 16, **dadurch gekennzeichnet, dass** sich die Leitschaufeln (52) in einem Radringbereich (66) des Radiallaufrades (38) erstrecken, der eine konstante Dicke, vorzugsweise von 4,5 mm bis 5 mm oder von 7 mm oder von 8 mm bis 9 mm, oder eine Dicke hat, die in Richtung nach radial außen konvergiert, vorzugsweise von 7,5 mm auf 2,5 mm oder von 13 mm auf 7 mm oder von 15 mm auf 8 mm oder 9 mmm.
